(19) **Europäisches Patentamt**

**European Patent Office**

**Office européen des brevets**

(11) **EP 0 728 831 B1**

(12) **EUROPÄISCHE PATENTSCHRIFT**

(45) Veröffentlichungstag und Bekanntmachung des Hinweises auf die Patenterteilung:
**12.07.2000  Patentblatt 2000/28**

(51) Int Cl.[7]: **C10G 11/00**

(21) Anmeldenummer: **96102178.9**

(22) Anmeldetag: **14.02.1996**

(54) **Verfahren zum Spalten von Kohlenwasserstoffen und Vorrichtung**

Process and apparatus for the cracking of hydrocarbons

Procédé et appareillage pour le craquage d'hydrocarbures

(84) Benannte Vertragsstaaten:
**BE DE DK ES FR GB IT NL PT SE**

(30) Priorität: **17.02.1995  DE 19505455**
**10.01.1996  DE 19600684**

(43) Veröffentlichungstag der Anmeldung:
**28.08.1996  Patentblatt 1996/35**

(73) Patentinhaber:
• **Linde Aktiengesellschaft**
**65189 Wiesbaden (DE)**
• **VEBA OEL AG**
**45896 Gelsenkirchen (DE)**

(72) Erfinder:
• **Kaufmann, Dieter, Dr.**
**D-82538 Geretsried (DE)**

• **Zimmermann, Heinz, Dr.**
**D-81476 München (DE)**
• **Wyrostek, Michael**
**D-45968 Gladbeck (DE)**

(74) Vertreter: **Kasseckert, Rainer**
**Linde Aktiengesellschaft,**
**Zentrale Patentabteilung**
**82049 Höllriegelskreuth (DE)**

(56) Entgegenhaltungen:
**EP-A- 0 662 505      DD-A- 243 647**
**DE-A- 2 830 824      DE-A- 2 923 596**
**FR-A- 2 192 162      FR-A- 2 198 003**

**Beschreibung**

Die Erfindung betrifft ein Verfahren zum Spalten von Kohlenwasserstoffen, von Kohlenwasserstoffgemischen oder von Kohlenwasserstoff-Fraktionen, wobei die Spaltung in zumindest teilweise mit Katalysator beschichteten Reaktionsrohren durchgeführt wird, und eine Vorrichtung mit zumindest teilweiser katalytischer Beschichtung.

Spaltverfahren können unterschiedlichen Zielsetzungen unterworfen sein. Das Steamcracken beispielsweise dient üblicherweise der Gewinnung von niederen Olefinen, insbesondere von Ethylen. Als Einsatz können in Ethylenanlagen Kohlenwasserstoffgemische von Ethan bis Gasöl eingesetzt werden. Es sind aber auch andere Verfahren zur Spaltung von Kohlenwasserstoffen wie beispielsweise die Dampfreformierung bekannt, die in der Regel im Zusammenhang mit der Herstellung von Synthesegas eingesetzt wird.

Bei der thermischen Spaltung von Kohlenwasserstoffen, insbesondere von schweren Kohlenwasserstoffen, werden bekanntermaßen Spaltöfen mit zumindest einer Konvektions- und Strahlungszone eingesetzt. Die thermische Spaltung wird dabei in der brennerbeheizten Strahlungszone durchgeführt, während in der Konvektionszone die Kohlenwasserstoffe und andere Fluide durch dort angeordnete Wärmetauscher gegen Rauchgas erhitzt werden. Üblicherweise werden Kohlenwasserstoffe und Prozeßdampf in den Wärmetauschern der Konvektionszone vorgewärmt und ein Kohlenwasserstoff/ Dampfgemisch den in der Strahlungszone angeordneten Rohrschlangen (coils) zugeführt. Die Spaltgase werden im nachgeordneten Quenchkühler zur Unterbrechung der Reaktionen rasch abgekühlt. Derartige Verfahren und Spaltöfen sind beispielsweise aus der DE-OS 28 30 824 und der DE-PS 28 54 061 bekannt.

Bei Spaltverfahren setzt sich Koks an unterschiedlichsten Anlagenteilen und Apparaten ab, beispielsweise in den Spaltrohren, aber auch in Quenchkühlern. Dies führt beispielsweise beim konventionellen Steamcracken dazu, daß meist in regelmäßigen Abständen entkokt werden muß. Die Entkokung bringt daher Produktionsausfallzeiten mit sich, die durch Reserveöfen kompensiert werden müssen.

Dies gilt im Prinzip für alle Kohlenwasserstoffeinsätze, wobei bei gleicher Spaltschärfe und Verweilzeit die Abstände zwischen zwei Entkokungen um so kürzer werden und damit die Gesamtausfallzeiten zunehmen, je schwerer der verwendete Kohlenwasserstoffeinsatz ist. Die Entkokungen bedeuten außerdem zusätzlichen Wartungsaufwand.

Aus der Patentschrift DD-243 708 A1 ist ein Verfahren zum thermokatalytischen Spalten von höhersiedenden Kohlenwasserstoffen bekannt. Dabei wird ein Vergasungskatalysator eingesetzt, der auf Calciumaluminatbasis aufgebaut ist und mit einem Alkalivanadat wie Kaliumpyrovanadat dotiert ist. Ein derartiger Vergasungskatalysator und ein Verfahren zu seiner Herstellung wird in der deutschen Patentschrift DD-243 647 A1 beschrieben.

Bei Verfahren zur Spaltung von Kohlenwasserstoffen in herkömmlichen Spaltrohren oder Spaltrohrschlangen können die Spaltrohre beispielsweise zur thermokatalytischen Spaltung zumindestens teilweise mit einem Vergasungskatalysator gefüllt werden. Es hat sich gezeigt, daß die Katalysatorfüllungen in den konventionellen Reaktionsrohrschlangen von Pyrolyseöfen oder von Steamcrackern eine Reihe von Nachteilen mit sich bringen. Durch die Katalysatorfüllungen in den Reaktionsrohren erhöht sich der Druckverlust der einzelnen Reaktionsrohrschlangen erheblich. Ferner ist das Eigengewicht der katalysatorgefüllten Reaktionsrohre gegenüber ungefüllten Reaktionsrohren deutlich vergrößert, wodurch die mechanische Belastung zunimmt. Der hohe Druckabfall in den Reaktionsrohren wiederum macht erforderlich, daß die Wandstärke der Reaktionsrohre erhöht werden muß, was sich negativ auf die Wärmeübertragung auswirkt. Darüber hinaus verringert sich durch das Volumen des Katalysators die Raum-Zeit-Ausbeute der Reaktionsschlangen, so daß neue Ofenkonzepte erforderlich würden. In der Druckschrift DE 44 00 430 A1 sind ein Verfahren zum thermokatalytischen Spalten von höhersiedenden Kohlenwasserstoffen und ein Spaltofen beschrieben, wobei den aufgrund der Katalysatorfüllungen auftretenden Problemen durch die Verwendung gerader Reaktionsrohre ohne Rohrkrümmer Rechnung getragen wird.

Die DE 29 23 596 A1 lehrt die thermische Umwandlung von Gasgemischen wie Kohlenwasserstoffen in Prozeßöfen, wobei mindestens auf einem geradlinigen Rohr mit zur Rohrachse symmetrischer innerer Gitterwandung (gitterartiger Innenaufbau) je nach Umwandlungsprozeß mit oder ohne Katalysator beschichtet ist, wobei im Gegensatz zu den üblicherweise verwendeten Prozeßrohren aus Metall Rohre aus Keramik eingesetzt werden.

Die FR 2 198 003 A1 beschreibt die Verwendung von beschichteten Trägermetallen beispielsweise in Form von Rohren für die thermische Spaltung von Kohlenwasserstoffgemischen, wobei die Trägermetalle aus Stahl oder einer hitzebeständigen Legierung bestehen und eine beim Kontaktieren mit organischen Substanzen bei erhöhten Temperaturen schützende und/ oder katalytische Aktivität aufweisende Schicht tragen. Die Schicht wird aus einem Metallpulver und/oder einer pulverförmigen Legierung hergestellt. Die Beschichtung soll verhindern, daß der Kohlenstoff auf den Innenwänden der Rohre in das Metall hineindiffundiert und die mechanischen Eigenschaften des Metalls (z.B. kurze Lebensdauer aufgrund Brüchigwerden) verändert.

Der Erfindung liegt die Aufgabe zugrunde, ein Verfahren und eine Vorrichtung der eingangs genannten Art aufzuzeigen, die die genannten Nachteile vermeiden und/oder Spaltverfahren insgesamt verbessern und gegebenenfalls vereinfachen können. Insbesondere sollte eine Spaltung ohne oder mit nur geringen Laufzeiteinbußen wegen entkokungsbedingter Ausfallzeiten bei einer hohen Spaltschärfe ermöglicht werden. Außerdem sollten die durch Katalysatorfüllungen in Spaltrohren auftretenden Probleme möglichst vermieden werden.

Diese der Erfindung zugrundeliegende Aufgabe wird für das erfindungsgemäße Verfahren dadurch gelöst, daß der Katalysator die Vergasung von Koks mit Wasserdampf nach der Wassergasreaktion in CO, $CO_2$ und $H_2$ und/oder die Spaltreaktionen fördert und auf Calciumaluminatbasis aufgebaut ist.

Die Beschichtung kann dabei unmittelbar auf der Innenoberfläche des Reaktionsrohres oder auf einem zuvor aufgetragenen Haftvermittler und/oder auf einem im Reaktionsrohr angeordneten Hilfsträger wie beispielsweise auf einem in der Rohrform vorgeformten Gitternetz aufgebracht sein. Die erfindungsgemäßen Katalysatorbeschichtungen erlauben prinzipiell die Verwendung aller bekannten Formen von Reaktionsrohren für die Spaltung. Sie führen außerdem zu keinem wesentlichen Druckverlust in den Reaktionsrohren. Dadurch, daß die Raum/Zeit-Ausbeute durch die Beschichtung nicht verändert wird, ist dieses Verfahren für den Einsatz in bestehenden Anlagen besonders attraktiv.

Der erfindungsgemäß als Beschichtung aufgetragene Katalysator kann die Vergasung von Koks mit Wasserdampf nach der Wassergasreaktion in CO, $CO_2$ und $H_2$ und/oder die Spaltreaktionen fördern. Die endotherme Wassergasreaktion läuft dabei nach der Reaktionsgleichung

$$C + H_2O \Leftrightarrow CO + H_2 \qquad (1)$$

und

$$CO + H_2O \Leftrightarrow CO_2 + H_2 \qquad (2)$$

ab. Unter anderem durch die Erzeugung und damit Bereitstellung von Wasserstoff wird die Spaltung in leichte Kohlenwasserstoffe unterstützt. Ein entsprechend ausgewählter Katalysator kann die Spaltreaktionen aber auch direkt fördern.

Die erfindungsgemäße Katalysatorbeschichtung kann durch jedes bekannte Auftragsverfahren, bevorzugt thermisch, mittels Kaltbeschichtungs-Verfahren wie insbesondere mittels des Schlickerverfahrens, mittels Aufdampf-Verfahren und/oder mittels Kleben aufgebracht werden.

Die thermischen Beschichtungsverfahren umfassen dabei insbesondere Flammspritz-, Flammschockspritz-, Hochgeschwindigkeitsflammspritz-, Plasmaspritz-, Lichtbogenspritz- und Schmelzbadspritzverfahren.

Beim Schlickerverfahren wird das Katalysatorpulver in eine wässrige Lösung gebracht und mit einem Bindemittel versetzt, wobei der Bindemittelanteil in der Regel kleiner als 10% bleibt, und schließlich als Suspension aufgebracht, beispielsweise durch Tauchen, Sprühen, Aufstreichen oder dergleichen. Diese so aufgetragene Schicht wird in der Regel noch thermisch nachbehandelt, beispielsweise getrocknet, eingebrannt und/oder gesintert. Als Bindemittel eigenen sich insbesondere Phosphate, Silikate und/oder Aluminate. Eine derartige Schlickerschicht kann auch auf eine thermisch gespritzte (beispielsweise durch Lichtbogenspritzen) Haftvermittlerschicht aufgebracht werden.

Unter die Aufdampfverfahren fallen die als PVD-Verfahren (**P**hysical **V**apour **D**eposition) und als CVD-Verfahren (**C**hemical **V**apour **D**eposition) bekannten Verfahren.

Wie bereits beschrieben kann der Katalysator die Vergasung von Koks mit Wasserdampf nach der Wassergasreaktion in CO, $CO_2$ und $H_2$ fördern und der bei der katalytischen Vergasung gewonnene Wasserstoff für die Spaltung der Kohlenwasserstoffe verwendet werden. Der gewonnene Wasserstoff kann aber auch mit Vorteil als zusätzliches Produkt zur Verfügung stehen, wie es beispielsweise bei der Synthesegasproduktion angestrebt wird.

Erfindungsgemäß wird ein auf Calciumaluminatbasis aufgebauter Katalysator, bevorzugt mit einer Dotierung von Alkalivanadat, insbesondere von Kaliumpyrovanadat, eingesetzt, weil er sich einerseits für die Wassersgasreaktion hervorragend eignet und außerdem mittels der beschriebenen Auftragsverfahren aufgebracht werden kann. Ein solcher Katalysator ist beispielsweise in der DD-243 708 A1, der DD-243 647 A1 oder der Patentanmeldung DE 44 00 430 A1 beschrieben. Die Vergasungsaktivität des Katalysators ist dabei durch seine Zusammensetzung bestimmt. Durch die Wahl einer bestimmten Zusammensetzung des Vergasungskatalysators kann die Vergasungsaktivität des Katalysators also an die Verkokungstendenz des Kohlenwasserstoff-Einsatzes angepaßt werden. Damit kann eine übermäßige Vergasung des Spalteinsatzes verhindert werden. Allerdings muß gewährleistet bleiben, daß die Vergasungsaktivität des Katalysators ausreicht, um Kohlenstoffablagerung und/oder Koks in den Reaktionsrohren zu unterbinden, oder auf ein unschädliches Maß zu beschränken. Untersuchungen haben überraschenderweise ergeben, daß beispielsweise bei der thermokatalytischen Spaltung eine hohe Vergasungsaktivität nicht zu einer Verringerung der Ausbeuten führt, sondern vielmehr bei einer hohen Vergasungsaktivität die Menge an Pyrolyseöl, d.h. die Produktfraktion mit einem Siedepunkt über ca. 200°C, deutlich abnimmt. Damit kann auf eine hydrierende Vorbehandlung der schweren Kohlenwasserstoffe unter dem erforderlichen hohen Druck verzichtet werden.

Besondere Vorteile sind im erfindungsgemäßen Verfahren zu erzielen, wenn die Spaltung aus einer Dampfspaltung, einer Pyrolyse, einer thermokatalytischen Spaltung oder einer Dampfreformierung besteht. Es hat sich gezeigt, daß bei Verwendung von beschichteten Reaktionsrohren beispielsweise in Steamcrakkern oder bei der thermokatalytischen Spaltung die Olefinausbeute des Spaltprodukts bei einer kurzen Verweilzeit besonders hoch ausfällt und eine gute Selektivität der Spaltung erzielt wird.

Die Einsatzqualität der Kohlenwasserstoff-Fraktionen wird üblicherweise durch den BMCI-Wert (Bureau of Mines Corellation Index) charakterisiert, der in etwa dem Gehalt an Aromaten des Kohlenwasserstoff-Einsatzes entspricht. Einsätze mit einem relativ niedrigen BMCI-Wert lassen sich also besonders gut spalten. Durch das erfindungsgemäße thermische Spaltverfahren können Kohlenwasserstoff-Einsätze sogar bis zu einem BMCI-Wert von etwa 60 rentabel verarbeitet werden.

Weitere Vorteile im erfindungsgemäßen Verfahren ergeben sich, wenn die Massengeschwindigkeit (Verhältnis der Masse des Kohlenwasserstoff-Einsatzes und des Dampfs pro Sekunde je Rohrquerschnittsfläche) einen Wert zwischen 5 und 300 $kg/s \cdot m^2$, vorzugsweise zwischen 10 und 200 $kg/s \cdot m^2$, besonders bevorzugt zwischen 20 und 120 $kg/s \cdot m^2$, aufweist.

In Ausgestaltung des erfindungsgemäßen Verfahrens bleibt die mittlere innere Heizflächenbelastung der Reaktionsrohre auf einen Wert zwischen 10 und 120 $kW/m^2$, vorzugsweise zwischen 15 und 80 $kW/m^2$, beschränkt. Bei einer derartigen Heizflächenbelastung der Reaktionsrohre ist eine hohe Wärmeübertragung oder ein hoher Wärmeeintrag auf das Kohlenwasserstoff/Dampfgemisch in den Reaktionsrohren gewährleistet, ohne daß die Reaktionsrohre über Gebühr beansprucht werden.

Vorteilhafterweise können die Spaltgase in mit Katalysator beschichteten Rohren eines Spaltgaskühlers rasch abgekühlt werden. Damit wird auch im Spaltgaskühler (z.B. TLE oder TLX) eine Verkokung unterbunden oder wesentlich eingeschränkt.

Die erfindungsgemäße Katalysatorbeschichtung kann auf einfache Art und Weise für die Nachrüstung von bestehenden Spaltanlagen verwendet werden. Bestehende Spaltanlagen können wirksam verbessert werden, indem die Beschichtung mit Katalysator nachträglich aufgebracht wird.

Ein erfindungsgemäßer Gegenstand, also ein Spaltofen, ein Dampfreformer, ein Reaktor, ein Quenchkühler, ein Verdampfer und/oder ein Wärmetauscher, weist zumindest teilweise eine katalytische Beschichtung auf, wobei Oberflächen, die beim Betrieb der Vorrichtung in Kontakt mit Kohlenstoff und/oder Kohlenstoffverbindungen, insbesondere Kohlenwasserstoffen, sind, insbesondere Rohre und/oder Wandflächen, zur Verringerung oder Vermeidung von Koksablagerungen erfindungsgemäß zumindest teilweise mit einem Katalysator zur Förderung der Vergasung von Koks und/oder zur Förderung der Spaltreaktionen beschichtet sind, der auf Calciumaluminatbasis aufgebaut ist. Derartige Vorrichtungen weisen wegen der nun entfallenden oder zumindest wesentlich verringerten Produktionsausfallzeiten eine erhöhte Anlagenkapazität auf. Zusätzlich kann durch die Erfindung der erforderliche Wartungsaufwand für die Vorrichtung reduziert werden.

Es besteht eine einfache Installationsmöglichkeit der Beschichtung an Reaktionsrohren und/oder Oberflächen. Dies gilt sowohl für den Bau von Neuanlagen als auch für den Umbau von bestehenden Anlagen. Die Beschichtung kann unmittelbar auf den Reaktionsrohren oder Oberflächen oder aber auf einem zuvor aufgetragenen Haftvermittler und/oder auf einem in den Reaktionsrohren oder auf den Oberflächen angeordneten Hilfsträger, insbesondere auf einem vorzugsweise in Rohrform vorgeformten Gitternetz, aufgebracht sein. Die Hilfsträger sind dabei den Reaktionsrohren respektive den Oberflächen zugeordnet. Die Beschichtung ist besonders leicht und dauerhaft aufzutragen, wenn die Oberflächen, die Reaktionsrohre und/oder die Hilfsträger eine thermische Beschichtung, eine Kaltbeschichtung, insbesondere eine Schlickerbeschichtung, eine Aufdampfbeschichtung und/oder eine Klebebeschichtung aufweisen, wobei gegebenenfalls die Katalysatorbeschichtung auf einer Haftvermittlerschicht aufgebracht sein kann.

Die Katalysatorbeschichtung ist erfindungsgemäß auf Calciumaluminatbasis aufgebaut. Einige die Wassergasreaktion fördernde Katalysatoren, die CaO, MgO und/oder kalzinierte Dolomiten umfassen, sind beispielsweise aus "Thermal and Catalytic Cracking of *n*-Heptane in Presence of CaO, MgO and Calcinated Dolomites", G. Taralas, V. Vassilatos, K. Sjöström und J. Delgado, The Canadian Journal Of Chemical Engineering, Volume 69, Dezember 1991, Seiten 1413 bis 1419, bekannt. Die oben beschriebene Beschichtung aus einem auf Calciumaluminatbasis aufgebauten Katalysator, kann bevorzugt mit einer Dotierung von Alkalivanadat, insbesondere von Kaliumpyrovanadat, versehen sein.

**Patentansprüche**

1. Verfahren zum Spalten von Kohlenwasserstoffen, von Kohlenwasserstoffgemischen oder von Kohlenwasserstoff-Fraktionen, wobei die Spaltung in zumindest teilweise mit Katalysator beschichteten Reaktionsrohren durchgeführt wird, **dadurch gekennzeichnet**, daß der Katalysator die Vergasung von Koks mit Wasserdampf nach der Wassergasreaktion in CO, $CO_2$ und $H_2$ und/oder die Spaltreaktionen fördert und auf Calciumaluminatbasis aufgebaut ist.

2. Verfahren nach Anspruch 1, dadurch gekennzeichnet, daß die Beschichtung unmittelbar auf dem Reaktionsrohr oder auf einem zuvor aufgetragenen Haftvermittler und/oder auf einem im Reaktionsrohr angeordneten Hilfsträger, insbesondere auf einem vorzugsweise in Rohrform vorgeformten Gitternetz, aufgebracht wird.

3. Verfahren nach einem der Ansprüche 1 oder 2, dadurch gekennzeichnet, daß die Beschichtung thermisch, durch Kaltbeschichtungs-Verfahren, insbesondere das Schlickerverfahren, durch Aufdampf-Verfahren und/oder durch Kleben aufgebracht wird.

4. Verfahren nach einem der Ansprüche 1 bis 3, dadurch gekennzeichnet, daß der Katalysator die Vergasung von Koks mit Wasserdampf nach der Wassergasreaktion in CO, $CO_2$ und $H_2$ bewirkt und daß der bei der katalytischen Vergasung gewonnene Wasserstoff für die Spaltung der Kohlenwasserstoffe und/oder als zusätzliches Produkt zur Verfügung steht.

5. Verfahren nach einem der Ansprüche 1 bis 4, dadurch gekennzeichnet, daß ein Katalysator mit einer Dotierung von Alkalivanadat, insbesondere von Kaliumpyrovanadat, eingesetzt wird.

6. Verfahren nach einem der Ansprüche 1 bis 5, dadurch gekennzeichnet, daß die Spaltung aus einer Dampfspaltung, einer Pyrolyse, einer thermokatalytischen Spaltung oder einer Dampfreformierung besteht.

7. Verfahren nach einem der Ansprüche 1 bis 6, dadurch gekennzeichnet, daß die Massengeschwindigkeit (Verhältnis der Masse des Kohlenwasserstoff-Einsatzes und des Dampfs pro Sekunde je Rohrquerschnittsfläche) zwischen 5 und 300 kg/s·m², vorzugsweise zwischen 10 und 200 kg/s·m², besonders bevorzugt zwischen 20 und 120 kg/s·m², beträgt.

8. Verfahren nach einem der Ansprüche 1 bis 7, dadurch gekennzeichnet, daß die mittlere innere Heizflächenbelastung der Reaktionsrohre auf einen Wert zwischen 10 und 120 kW/m², vorzugsweise zwischen 15 und 80 kW/m², beschränkt bleibt.

9. Verfahren nach einem der Ansprüche 1 bis 8, dadurch gekennzeichnet, daß die Spaltgase in mit Katalysator beschichteten Rohren eines Spaltgaskühlers und/oder Quenchkühlers rasch abgekühlt werden.

10. Verfahren nach einem der Ansprüche 1 bis 9, dadurch gekennzeichnet, daß bestehende Spaltanlagen verbessert werden, indem die Beschichtung mit Katalysator nachträglich aufgebracht wird.

11. Spaltofen, Dampfreformer, Reaktor, Spaltgaskühler, Quenchkühler, Verdampfer und/oder Wärmetauscher mit zumindest teilweiser katalytischer Beschichtung, **dadurch gekennzeichnet,** daß Oberflächen, die beim Betrieb der Vorrichtung in Kontakt mit Kohlenstoff und/oder Kohlenstoffverbindungen, insbesondere Kohlenwasserstoffen, sind, insbesondere Rohre, zur Verhinderung oder Vermeidung von Koksablagerungen zumindest teilweise mit einem Katalysator zur Förderung der Vergasung von Koks mit Wasserdampf nach der Wassergasreaktion in CO, $CO_2$ und $H_2$ und/oder der Spaltreaktionen beschichtet sind, welcher auf Calciumaluminatbasis aufgebaut ist.

12. Vorrichtung nach Anspruch 12, dadurch gekennzeichnet, daß die Beschichtung unmittelbar auf den Reaktionsrohren oder Oberflächen oder auf einem zuvor aufgetragenen Haftvermittler und/oder auf einem in den Reaktionsrohren oder auf den Oberflächen angeordneten Hilfsträger, insbesondere auf einem vorzugsweise in Rohrform vorgeformten Gitternetz, aufgebracht sind.

13. Vorrichtung nach einem der Ansprüche 12 oder 13, dadurch gekennzeichnet, daß die Oberflächen, Reaktionsrohre und/oder die Hilfsträger eine thermische Beschichtung, eine Kaltbeschichtung, insbesondere eine Schlickerbeschichtung, eine Aufdampfbeschichtung und/oder eine Klebebeschichtung aufweisen, wobei gegebenenfalls die Katalysatorbeschichtung auf einer Haftvermittlerschicht aufgebracht ist.

14. Vorrichtung nach einem der Ansprüche 11 bis 13, dadurch gekennzeichnet, daß die Beschichtung aus einem Katalysator mit einer Dotierung von Alkalivanadat, insbesondere von Kaliumpyrovanadat, besteht.

**Claims**

1. Process for cleaving hydrocarbons, hydrocarbon mixtures or hydrocarbon fractions in which the cleavage is carried out in reaction tubes which are at least partially coated with catalyst, characterized in that the catalyst promotes the gasification of coke with steam into CO, $CO_2$ and $H_2$ in accordance with the water/gas reaction and/or promotes the cleavage reactions and is made up on a calcium aluminate base.

2. Process according to Claim 1, characterized in that the coating is applied directly to the reaction tube or to an adhesion promoter applied in advance and/or to an auxiliary support disposed in the reaction tube, in particular to a grid preferably preshaped into a tubular form.

3. Process according to one of Claims 1 or 2, characterized in that the coating is applied thermally, by cold-coating processes, in particular the slip process, by sputtering processes and/or by adhesion.

4. Process according to one of Claims 1 to 3, characterized in that the catalyst effects the gasification of coke with steam into CO, $CO_2$ and $H_2$ in accordance with the water-gas reaction and in that the hydrogen produced in the catalytic gasification is available for cleaving the hydrocarbons and/or as an additional product.

5. Process according to one of Claims 1 to 4, characterized in that a catalyst having a doping of alkali metal vanadate, in particular potassium pyrovanadate, is used.

6. Process according to one of Claims 1 to 5, characterized in that the cleavage consists of a steam cleavage, a pyrolysis, a thermocatalytic cleavage or a steam reformation.

7. Process according to one of Claims 1 to 6, characterized in that the mass velocity (ratio of the mass of hydrocarbon feed and steam per second per tube cross-sectional area) is between 5 and 300 kg/s·$m^2$, preferably between 10 and 200 kg/s·$m^2$, particularly preferably between 20 and 120 kg/s·$m^2$.

8. Process according to one of Claims 1 to 7, characterized in that the mean internal surface power density of the reaction tubes is restricted to a value between 10 and 120 kW/$m^2$, preferably between 15 and 80 kW/$m^2$.

9. Process according to one of Claims 1 to 8, characterized in that the cleavage gases are rapidly cooled in catalyst-coated tubes of a cleavage gas cooler and/or quench cooler.

10. Process according to one of Claims 1 to 9, characterized in that existing cleavage units are improved by subsequently applying the catalyst-containing coating.

11. Cleavage furnace, steam reformer, reactor, cleavage gas cooler, quench cooler, evaporator and/or heat exchanger having at least partial catalytic coating, characterized in that surfaces which, on operation of the apparatus, are in contact with carbon and/or carbon compounds, in particular hydrocarbons, in particular tubes, to impede or prevent coke depositions, are at least partially coated with a catalyst to promote the gasification of coke and steam into CO, $CO_2$ and $H_2$ in accordance with the water-gas reaction and/or to promote the cleavage reactions, which catalyst is made up of a calcium aluminate base.

12. Apparatus according to Claim 12, characterized in that the coating is applied directly to the reaction tubes or surfaces or to an adhesion promoter applied in advance and/or to an auxiliary support which is disposed in the reaction tubes or on the surfaces, in particular to a grid preferably preformed into a tubular shape.

13. Apparatus according to one of Claims 12 or 13, characterized in that the surfaces, reaction tubes and/or the auxiliary supports have a thermal coating, a cold coating, in particular a slip coating, a sputtered coating and/or an adhesive coating, if appropriate the catalyst coating being applied to an adhesion promoter layer.

14. Apparatus according to one of Claims 11 to 13, characterized in that the coating consists of a catalyst having a doping of alkali metal vanadate, in particular potassium pyrovanadate.

**Revendications**

1. Procédé pour le craquage d'hydrocarbures, de mélanges d'hydrocarbures ou de fractions d'hydrocarbure, le craquage étant effectué au moins partiellement avec des tubes à réaction revêtus de catalyseur, caractérisé en ce que le catalyseur favorise la gazéification du coke avec de la vapeur d'eau selon la réaction du gaz à l'eau, en CO, en $CO_2$ et en $H_2$ et/ou les réactions de craquage et est à base d'aluminate de calcium.

2. Procédé suivant la revendication 1, caractérisé en ce que le revêtement est appliqué immédiatement sur le tube à réaction ou sur un agent adhésif appliqué au préalable et/ou un support auxiliaire disposé dans le tube à réaction, en particulier sur un réseau grillagé préformé de préférence en forme de tube.

3. Procédé suivant l'une des revendications 1 ou 2, caractérisé en ce que le revêtement est appliqué de manière thermique, par un procédé de revêtement à froid, en particulier par le procédé à la barbotine, par un procédé d'application par évaporation et/ou par collage.

4. Procédé suivant l'une des revendications 1 à 3, caractérisé en ce que le catalyseur opère la gazéification du coke avec de la vapeur d'eau selon la réaction du gaz à l'eau, en CO, en $CO_2$ et en $H_2$ et en ce que l'hydrogène obtenu lors de la gazéification catalytique est disponible pour le craquage des hydrocarbures et/ou comme produit supplémentaire.

**5.** Procédé suivant l'une des revendications 1 à 4, caractérisé en ce qu'on utilise un catalyseur avec un dopage à un vanadate alcalin, en particulier au pyrovanadate de potassium.

**6.** Procédé suivant l'une des revendications 1 à 5, caractérisé en ce que le craquage est constitué d'un vapocraquage, d'une pyrolyse, d'un craquage thermocatalytique ou d'un reformage à la vapeur.

**7.** Procédé suivant l'une des revendications 1 à 6, caractérisé en ce que la vitesse massique (rapport de la masse de la charge d'hydrocarbure et de la vapeur par seconde à la surface de section de tube) se situe entre 5 et 300 kg/s·m$^2$, de préférence entre 10 et 200 kg/s·m$^2$, de manière particulièrement préférée entre 20 et 120 kg/s·m$^2$.

**8.** Procédé suivant l'une des revendications 1 à 7, caractérisé en ce que la charge calorifique intérieure moyenne par unité de surface de chauffe du tube à réaction reste limitée à une valeur entre 10 et 120 kW/m$^2$, de préférence entre 15 et 80 kW/m$^2$.

**9.** Procédé suivant l'une des revendications 1 à 8, caractérisé en ce que les gaz de craquage sont rapidement refroidis dans des tubes revêtus d'un catalyseur d'un refroidisseur de gaz de craquage et/ou d'un refroidisseur à injection.

**10.** Procédé suivant l'une des revendications 1 à 9, caractérisé en ce qu'on améliore les installations de craquage existantes en appliquant ultérieurement le revêtement de catalyseur.

**11.** Four à craquage, reformeur à la vapeur, réacteur, refroidisseur de gaz de craquage, refroidisseur à injection, évaporateur et/ou échangeur de chaleur avec un revêtement catalytique au moins partiel, caractérisés en ce que les surfaces, en particulier des tubes, qui sont en contact avec le carbone et/ou les composés carbonés, en particulier les hydrocarbures lors du fonctionnement de l'appareil, sont revêtues au moins partiellement d'un catalyseur, qui est à base d'aluminate de calcium, pour favoriser la gazéification du coke avec de la vapeur d'eau selon la réaction du gaz à l'eau, en CO, en CO$_2$ et en H$_2$ et/ou des réactions de craquage, pour empêcher ou éviter des dépôts de coke.

**12.** Appareillage suivant la revendication 12, caractérisé en ce que le revêtement est appliqué immédiatement sur les tubes à réaction ou les surfaces ou sur un agent adhésif appliqué au préalable et/ou sur un support auxiliaire disposé dans les tubes à réaction ou sur les surfaces, en particulier sur un réseau grillagé préformé de préférence en forme de tube.

**13.** Appareillage suivant l'une des revendications 12 ou 13, caractérisé en ce que les surfaces, les tubes à réaction et/ou les supports auxiliaires présentent un revêtement à chaud, un revêtement à froid, en particulier un revêtement à la barbotine, un revêtement appliqué par évaporation et/ou un revêtement par collage, le revêtement de catalyseur étant le cas échéant appliqué sur une couche d'agent adhésif.

**14.** Appareil suivant l'une des revendications 11 à 13, caractérisé en ce que le revêtement est constitué d'un catalyseur avec un dopage à un vanadate alcalin, en particulier au pyrovanadate de potassium.